# EUROPÄISCHE PATENTSCHRIFT

(11) **EP 2 731 929 B1**
(45) Veröffentlichungstag und Bekanntmachung des Hinweises auf die Patenterteilung: **05.10.2016**
(21) Anmeldenummer: 12737207.6
(22) Anmeldetag: 11.07.2012
(51) Int. Cl.: C07C 231/02, A61K 8/44, A61Q 19/00

(54) **VERFAHREN ZUR HERSTELLUNG VON ACYLGLYCINATEN UND ZUSAMMENSETZUNGEN ENTHALTEND DERARTIGE VERBINDUNGEN**
PROCESS FOR PREPARING ACYLGLYCINATES AND COMPOSITIONS COMPRISING SUCH COMPOUNDS
PROCÉDÉ DE PRÉPARATION D'ACYLGLYCINATES ET COMPOSITIONS CONTENANT DE TELS COMPOSÉS

(30) Priorität: 15.07.2011 DE 102011107503
(43) Veröffentlichungstag der Anmeldung: 21.05.2014
(73) Patentinhaber: Clariant International Ltd, 4132 Muttenz (CH)
(72) Erfinder: KLUG, Peter, 63762 Großostheim (DE); SCHERL, Franz-Xaver, 84508 Burgkirchen (DE)
(74) Vertreter: Holmes, Rosalind
(86) Internationale Anmeldenummer: PCT/EP2012/002931
(87) Internationale Veröffentlichungsnummer: WO 2013/010650

(56) Entgegenhaltungen:
- WO-A2-02/057217
- WO-A2-2009/065530

## Beschreibung

Die Erfindung betrifft ein Verfahren zur Herstellung von Acylglycinaten, Zusammensetzungen enthaltend Acylglycinate sowie deren Verwendung zur Herstellung von kosmetischen Zubereitungen oder als Tensid in kosmetischen Zubereitungen.

Acylglycinate wie z. B. Cocoylglycinate mit Na⁺ oder K⁺ als Gegenionen sind Tenside, die besonders in der Kosmetik für Gesichtsreinigungsrezepturen in Asien in Hautreinigungsprodukten geschätzt werden. Die Tenside, insbesondere das Natrium- und das Kaliumcocoylglycinat, schäumen in leicht alkalischer Lösung exzellent und erzeugen ein angenehmes, nicht öliges Hautgefühl.

Im Gegensatz zu den entsprechenden N-Methylglycinderivaten, den sogenannten Sarkosinaten, weisen Glycinate bei der Herstellung jedoch problematische Eigenschaften auf. Wie in JP 8053693 (Ajinomoto) beschrieben, lassen sich Acylglycinate in Wasser ohne zusätzliche Lösungsmittel zwar in Reinheiten von knapp über 90 % erhalten, bilden aber bei der Schotten-Baumann-Reaktion sehr hochviskose Reaktionslösungen. Die JP 8053693 schlägt bei der Herstellung von Acylglycinaten die Zugabe von Alkoholen wie Isopropanol, Isobutanol oder tert.-Butanol vor.

Diese Vorgehensweise ist jedoch wegen des Geruchs der Alkohole nicht vorteilhaft, weshalb die Alkohole aus der Reaktionsmischung auch nach Ansäuern und Phasentrennung wieder entfernt werden und das gewünschte Alkanoylglycinat nach Neutralisation meist als salzarme Qualität erhalten wird. Dieses Verfahren ist aufwändig und verursacht kochsalzhaltige Abwässer, die entsorgt werden müssen.

Die WO 2009/065530 (Clariant) beschreibt ein Verfahren zur Herstellung von Acylglycinaten der Formel R¹-NH-CH₂-COO⁻X⁺, worin R¹ für eine lineare oder verzweigte, gesättigte Alkanoylgruppe mit 6 bis 30 Kohlenstoffatomen oder für eine lineare oder verzweigte, ein- oder mehrfach ungesättigte Alkenoylgruppe mit 6 bis 30 Kohlenstoffatomen steht, und Q⁺ für ein Kation ausgewählt aus den Alkalimetallen Li⁺, Na⁺ und K⁺ steht, wobei Glycin mit Fettsäurechlorid R¹Cl in Wasser und in Gegenwart einer basischen Alkaliverbindung, die Kationen Q⁺ ausgewählt aus Li⁺, Na⁺ und K⁺ liefert, aber in Abwesenheit von organischen Lösungsmitteln, bei 30 bis 35 °C umgesetzt wird, und der Anteil an Fettsäurechlorid R¹Cl enthaltend Acylgruppen R¹ mit 18 oder mehr Kohlenstoffatomen, bezogen auf die Gesamtmenge an eingesetztem Fettsäurechlorid, kleiner als 2,0 Gew.-% ist. Des Weiteren werden in der WO 2009/065530 auch bestimmte Zusammensetzungen beschrieben enthaltend spezielle Acylglycinate und Wasser aber keine organischen Lösungsmittel sowie deren Verwendung zur Herstellung kosmetischer Zubereitungen oder als Tensid in kosmetischen Zubereitungen. Nachteil des in der WO 2009/065530 beschriebenen Verfahrens ist allerdings, dass keine preiswerten Standard-Fettsäurekettenschnitte Verwendung finden und deshalb noch ökonomische Verbesserungsmöglichkeiten bestehen. Zudem ist das Schaumverhalten der resultierenden Tenside bzw. Zusammensetzungen noch weiter verbesserungswürdig, insbesondere die erzielbaren Gesamtschaumhöhen.

Die WO 02/057217 (Cognis) offenbart ein Verfahren zur Herstellung von Acylaminosäuren, bei dem man in einem Reaktor eine Mischung aus mindestens einer Aminosäure oder deren Salz und einer Alkaliquelle vorlegt und diese in einem Mischelement mit Fettsäurehalogeniden der Formel R¹COX, worin R¹ für einen Alkyl- oder Alkenylrest mit 6 bis 22 Kohlenstoffatomen und X für Chlor, Brom, Jod steht, versetzt, die daraus resultierenden Produkte, sowie deren Verwendung in kosmetischen Produkten sowie in Wasch-, Spül- und Reinigungsmitteln. In den Beispielen gemäß der Lehre der WO 02/057217 wird die Herstellung in Gegenwart von organischen Lösungsmitteln durchgeführt.

Es bestand daher die Aufgabe, ein Verfahren zur Herstellung von Acylglycinaten bzw. von Zusammensetzungen enthaltend Acylglycinate zur Verfügung zu stellen, das die oben genannten Nachteile nicht aufweist oder diese Nachteile zumindest abschwächt und insbesondere den Vorteil aufweist, dass es ohne Einsatz von organischen Lösungsmitteln unter Verwendung preisgünstiger und marktgängiger Kettenschnitte der Fettsäurekomponente erfolgen kann und Acylglycinate von hoher Reinheit und mit vorteilhaftem Schaumvermögen wie z. B. vorteilhaften erzielbaren Gesamtschaumhöhen liefert. Das Verfahren sollte auch die direkte Herstellung von Zusammensetzungen mit hohem Aktivgehalt an Acylglycinat und mit niedrigen Viskositäten ermöglichen.

Weiterhin Aufgabe war es auch, entsprechende Zusammensetzungen enthaltend Acylglycinate zur Verfügung zu stellen. Diese Zusammensetzungen sollten daher beispielsweise folgende Vorteile aufweisen: Sie sollten das Acylglycinat in hoher Reinheit enthalten, ein vorteilhaftes Schaumvermögen besitzen und insbesondere vorteilhafte erzielbare Gesamtschaumhöhen ermöglichen. Die Zusammensetzungen sollten niedrige Viskositäten besitzen und gleichzeitig das Acylglycinat mit hohem Aktivgehalt enthalten. Sie sollten zudem keine organischen Lösungsmittel enthalten. Es sollte auch möglich sein, die in den Zusammensetzungen enthaltenen Acylglycinate oder die Zusammensetzungen selbst mit preisgünstigen und marktgängigen Kettenschnitten der Fettsäurekomponente herzustellen.

Überraschenderweise wurde gefunden, dass die Aufgabe gelöst wird, wenn Acylglycinate der Formel (I) 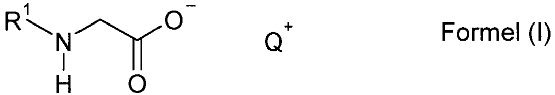 worin
- R¹: für eine lineare oder verzweigte, gesättigte Alkanoylgruppe mit 6 bis 30, vorzugsweise 8 bis 22 und besonders bevorzugt 8 bis 18 Kohlenstoffatomen, oder für eine lineare oder verzweigte, ein- oder mehrfach ungesättigte Alkenoylgruppe mit 6 bis 30, vorzugsweise 8 bis 22 und besonders bevorzugt 8 bis 18 Kohlenstoffatomen, steht, und
- Q⁺: für ein Kation ausgewählt aus den Alkalimetallkationen Na⁺ und K⁺ und vorzugsweise für Na⁺ steht,
durch Umsetzung von Glycin mit Fettsäurechlorid R¹Cl, wobei R¹ die in Formel (I) angegebene Bedeutung besitzt,
und für eine Acylgruppe mit 6 bis 30, vorzugsweise 8 bis 22 und besonders bevorzugt 8 bis 18 Kohlenstoffatomen steht, wobei
der Anteil an Fettsäurechloriden mit gesättigten C₈-Acylgruppen 5,0 - 8,0 Gew.-% ist,
der Anteil an Fettsäurechloriden mit gesättigten C₁₀-Acylgruppen 5,0 - 8,0 Gew.-% ist,
der Anteil an Fettsäurechloriden mit C₁₂-Acylgruppen 44,0 - 50,0 Gew.-% ist,
der Anteil an Fettsäurechloriden mit C₁₄-Acylgruppen 14,0 - 20,0 Gew.-% ist,
der Anteil an Fettsäurechloriden mit C₁₆-Acylgruppen 8,0 - 10,0 Gew.-% ist,
der Anteil an Fettsäurechloriden mit gesättigten C₁₈-Acylgruppen 1,0 - 3,0 Gew.-% ist, und
der Anteil an Fettsäurechloriden mit ungesättigten C₁₈-Acylgruppen 4,0 - 10,0 Gew.-% ist,
jeweils bezogen auf die Gesamtmenge an eingesetztem Fettsäurechlorid,
in Wasser und in Gegenwart einer basischen Alkaliverbindung, die Kationen Q⁺ ausgewählt aus Na⁺ und K⁺ und vorzugsweise Na⁺ liefert, aber in Abwesenheit von organischen Lösungsmitteln, bei einer Temperatur von 25 - 50 °C, bevorzugt bei einer Temperatur von 30 - 40 °C, hergestellt werden.

Gegenstand der Erfindung ist daher ein Verfahren zur Herstellung von Acylglycinaten der Formel (I) 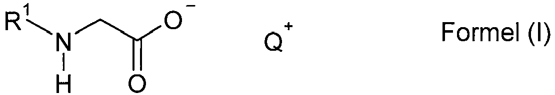 worin
- R¹: für eine lineare oder verzweigte, gesättigte Alkanoylgruppe mit 6 bis 30, vorzugsweise 8 bis 22 und besonders bevorzugt 8 bis 18 Kohlenstoffatomen, oder für eine lineare oder verzweigte, ein- oder mehrfach ungesättigte Alkenoylgruppe mit 6 bis 30, vorzugsweise 8 bis 22 und besonders bevorzugt 8 bis 18 Kohlenstoffatomen, steht, und
- Q⁺: für ein Kation ausgewählt aus den Alkalimetallkationen Na⁺ und K⁺ und vorzugsweise für Na⁺ steht,
dadurch gekennzeichnet, dass Glycin mit Fettsäurechlorid R¹Cl, wobei R¹ die in Formel (I) angegebene Bedeutung besitzt,
und für eine Acylgruppe mit 6 bis 30, vorzugsweise 8 bis 22 und besonders bevorzugt 8 bis 18 Kohlenstoffatomen steht, wobei
der Anteil an Fettsäurechloriden mit gesättigten C₈-Acylgruppen 5,0 - 8,0 Gew.-% ist,
der Anteil an Fettsäurechloriden mit gesättigten C₁₀-Acylgruppen 5,0 - 8,0 Gew.-% ist,
der Anteil an Fettsäurechloriden mit C₁₂-Acylgruppen 44,0 - 50,0 Gew.-% ist,
der Anteil an Fettsäurechloriden mit C₁₄-Acylgruppen 14,0 - 20,0 Gew.-% ist,
der Anteil an Fettsäurechloriden mit C₁₆-Acylgruppen 8,0 - 10,0 Gew.-% ist,
der Anteil an Fettsäurechloriden mit gesättigten C₁₈-Acylgruppen 1,0 - 3,0 Gew.-% ist, und
der Anteil an Fettsäurechloriden mit ungesättigten C₁₈-Acylgruppen 4,0 - 10,0 Gew.-% ist,
jeweils bezogen auf die Gesamtmenge an eingesetztem Fettsäurechlorid,
in Wasser und in Gegenwart einer basischen Alkaliverbindung, die Kationen Q⁺ ausgewählt aus Na⁺ und K⁺ und vorzugsweise Na⁺ liefert, aber in Abwesenheit von organischen Lösungsmitteln, bei 25 - 50 °C, vorzugsweise bei 30 - 40 °C, umgesetzt wird.

Im Rahmen der vorliegenden Erfindung werden die linearen oder verzweigten, gesättigten Alkanoylgruppen R¹ aus Formel (I) und die linearen oder verzweigten, ein- oder mehrfach ungesättigten Alkenoylgruppen R¹ aus Formel (I) zusammen auch als "Acylgruppen" bezeichnet.

Durch das erfindungsgemäße Verfahren erhält man Zusammensetzungen, die bei 40 °C als einphasige Zusammensetzungen vorliegen und sich deshalb für einen ökonomischen Transport eignen.

Die im erfindungsgemäßen Verfahren verwendeten Fettsäurechloride können nach dem Fachmann bekannten Methoden erhalten werden, z. B. durch Chlorierung von Fettsäuren wie z. B. handelsüblicher Kokosfettsäure mittels Phosphortrichlorid, Thionylchlorid oder Phosgen.

Als basische Alkaliverbindungen werden vorzugsweise Carbonate oder Hydroxide der Alkalimetallkationen Na⁺ oder K⁺, d.h. Na₂CO₃, NaOH, K₂CO₃ oder KOH, oder Mischungen davon eingesetzt. Besonders bevorzugt sind Na₂CO₃ oder NaOH oder Mischungen davon und insbesondere bevorzugt ist NaOH.

Das erfindungsgemäße Verfahren wird vorzugsweise bei einem pH-Wert von 9 bis 13, besonders bevorzugt 12 bis 13, durchgeführt.

Weiterhin bevorzugt wird das erfindungsgemäße Verfahren derart durchgeführt, dass Glycin und Fettsäurechlorid R¹Cl in annähernd äquimolaren Mengen eingesetzt werden. Besonders bevorzugt wird das Fettsäurechlorid R¹Cl bezogen auf Glycin in äquimolarer Menge oder in leichtem Unterschuss eingesetzt. Das molare Verhältnis von Glycin zu Fettsäurechlorid R¹Cl ist insbesondere bevorzugt von 1,1 : 1,0 bis 1,0 : 1,0 und außerordentlich bevorzugt von 1,05 : 1,0 bis 1,0 : 1,0.

Weiterhin werden in dem erfindungsgemäßen Verfahren Fettsäurechloride R¹Cl, worin R¹ für eine Acylgruppe mit 6 bis 30, vorzugsweise 8 bis 22 und besonders bevorzugt 8 bis 18 Kohlenstoffatomen steht, wobei
der Anteil an Fettsäurechloriden mit gesättigten C₈-Acylgruppen 5,0 - 8,0 Gew.-% ist,
der Anteil an Fettsäurechloriden mit gesättigten C₁₀-Acylgruppen 5,0 - 8,0 Gew.-% ist,
der Anteil an Fettsäurechloriden mit C₁₂-Acylgruppen 44,0 - 50,0 Gew.-% ist, der Anteil an Fettsäurechloriden mit C₁₄-Acylgruppen 14,0 - 20,0 Gew.-% ist, der Anteil an Fettsäurechloriden mit C₁₆-Acylgruppen 8,0 - 10,0 Gew.-% ist, der Anteil an Fettsäurechloriden mit gesättigten C₁₈-Acylgruppen 1,0 - 3,0 Gew.-% ist und
der Anteil an Fettsäurechloriden mit ungesättigten C₁₈-Acylgruppen, vorzugsweise Ölsäurechlorid, 4,0 - 10,0 Gew.-% ist,
jeweils bezogen auf die Gesamtmenge an eingesetztem Fettsäurechlorid, umgesetzt. Vorzugsweise findet diese Umsetzung in Gegenwart einer basischen Alkaliverbindung, die Na⁺-Kationen liefert, statt. Bei den basischen Alkaliverbindungen handelt es sich dabei vorzugsweise um Na₂CO₃ oder NaOH oder um Mischungen davon und besonders bevorzugt um NaOH.

Die Fettsäurechloride sind vorzugsweise linear.

Die Fettsäurechloride mit 12, 14 und 16 Kohlenstoffatomen sind vorzugsweise gesättigt.

Vorzugsweise ist der Anteil an Fettsäurechlorid mit 8 bis 18 Kohlenstoffatomen, bezogen auf die Gesamtmenge an eingesetztem Fettsäurechlorid, 95 Gew.-% oder größer und besonders bevorzugt 96,5 Gew.-% oder größer.

Weiterhin bevorzugt werden in dem erfindungsgemäßen Verfahren Cocoylchloride umgesetzt, wobei der Cocoylschnitt einer natürlichen Kokosfettsäure entspricht.

Das erfindungsgemäße Verfahren wird vorzugsweise derart ausgeführt, dass Glycin in Wasser in Gegenwart der basischen Alkaliverbindung vorgelegt wird und das Fettsäurechlorid bei 25 bis 50 °C und bevorzugt bei 30 bis 40 °C zugegeben wird. Die Zugabe des Fettsäurechlorids erfolgt dabei vorzugsweise langsam unter Rühren.

Durch das erfindungsgemäße Verfahren können hoch konzentrierte, salzhaltige Glycinatlösungen mit niedrigem Gehalt an Nebenprodukten (wie beispielsweise Fettsäuresalz) hergestellt werden, die bei 40 °C einphasig und niedrigviskos und dementsprechend einfach zu handhaben sind und darüber hinaus kosteneffektiv sind, da kein Separationsschritt durchgeführt werden muss. Außerdem wird dabei kein organisches Reaktionslösungsmittel benötigt, das wieder abgetrennt und gegebenenfalls entsorgt werden muss.

Weiterer Gegenstand der Erfindung sind daher Zusammensetzungen enthaltend
a) ein oder mehrere Acylglycinate der Formel (I) 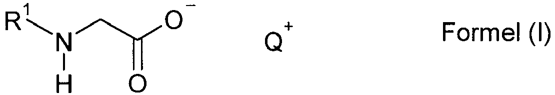 worin
   - R¹: für eine lineare oder verzweigte, gesättigte Alkanoylgruppe mit 6 bis 30, vorzugsweise 8 bis 22 und besonders bevorzugt 8 bis 18 Kohlenstoffatomen, oder für eine lineare oder verzweigte, ein- oder mehrfach ungesättigte Alkenoylgruppe mit 6 bis 30, vorzugsweise 8 bis 22 und besonders bevorzugt 8 bis 18 Kohlenstoffatomen, steht, und
   - Q⁺: für ein Kation ausgewählt aus den Alkalimetallkationen Na⁺ und K⁺ und vorzugsweise für Na⁺ steht,
   in Mengen von 21,0 - 28,0 Gew.-% und vorzugsweise in Mengen von 23,0 - 27,0 Gew.-%, bezogen auf die gesamte Zusammensetzung,
   und wobei in dem einen oder den mehreren Acylglycinaten der Formel (I)
   der Anteil an gesättigten C₈-Acylgruppen 5,0 - 8,0 Gew.-% ist,
   der Anteil an gesättigten C₁₀-Acylgruppen 5,0 - 8,0 Gew.-% ist,
   der Anteil an C₁₂-Acylgruppen 44,0 - 50,0 Gew.-% ist,
   der Anteil an C₁₄-Acylgruppen 14,0 - 20,0 Gew.-% ist,
   der Anteil an C₁₆-Acylgruppen 8,0 - 10,0 Gew.-% ist,
   der Anteil an gesättigten C₁₈-Acylgruppen 1,0 - 3,0 Gew.-% ist und der Anteil an ungesättigten C₁₈-Acylgruppen 4,0 - 10,0 Gew.-% ist, und wobei die genannten Anteile auf die Gesamtmenge der in dem einen oder den mehreren Acylglycinaten enthaltenen Acylgruppen bezogen sind, aber nicht auf Basis der Acylgruppen selbst, sondern auf Basis der zu den Acylgruppen korrespondierenden Fettsäurechloride berechnet werden,
b) ein oder mehrere Substanzen Q⁺Cl⁻, worin Q⁺ die Bedeutung von Q⁺ aus Formel (I) hat, in Mengen größer oder gleich 1,0 Gew.-%, vorzugsweise in Mengen von 1,0 bis 8,0 Gew.-%, besonders bevorzugt in Mengen von 2,0 bis 7,0 Gew.-% und insbesondere bevorzugt in Mengen von 4,0 bis 6,0 Gew.-%, bezogen auf die gesamte Zusammensetzung,
c) ein oder mehrere Fettsäuresalze der Formel (II) 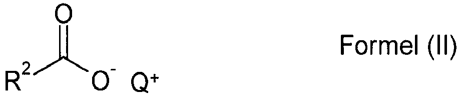 worin
   - R²CO: die Bedeutung von R¹ aus Formel (I) und
   - Q⁺: die Bedeutung von Q⁺ aus Formel (I) hat,
   in Mengen kleiner oder gleich 3,0 Gew.-%, vorzugsweise in Mengen von 0,01 bis 2,0 Gew.-%, besonders bevorzugt in Mengen von 0,1 bis 2,0 Gew.-% und insbesondere bevorzugt in Mengen von 0,1 bis 1,5 Gew.-%, bezogen auf die gesamte Zusammensetzung, und
d) Wasser,
e) aber keine organischen Lösungsmittel.

Die erfindungsgemäßen Zusammensetzungen ermöglichen vorteilhafte erzielbare Gesamtschaumhöhen und zudem gute Schaumstabilitäten.

Die erfindungsgemäßen Zusammensetzungen erhalten als Komponente a) ein oder mehrere Acylglycinate der Formel (I), worin R¹ für eine Acylgruppe mit 6 bis 30, vorzugsweise 8 bis 22 und besonders bevorzugt 8 bis 18 Kohlenstoffatomen steht, und Q⁺ für ein Kation ausgewählt aus Na⁺ und K⁺ und vorzugsweise für Na⁺ steht, in Mengen von 21,0 - 28,0 Gew.-% und vorzugsweise in Mengen von 23,0 - 27,0 Gew.-%, bezogen auf die gesamte Zusammensetzung, und wobei in dem einen oder den mehreren Acylglycinaten der Formel (I)
der Anteil an gesättigten C₈-Acylgruppen 5,0 - 8,0 Gew.-% ist,
der Anteil an gesättigten C₁₀-Acylgruppen 5,0 - 8,0 Gew.-% ist,
der Anteil an C₁₂-Acylgruppen 44,0 - 50,0 Gew.-% ist,
der Anteil an C₁₄-Acylgruppen 14,0 - 20,0 Gew.-% ist,
der Anteil an C₁₆-Acylgruppen 8,0 - 10,0 Gew.-% ist,
der Anteil an gesättigten C₁₈-Acylgruppen 1,0 - 3,0 Gew.-% ist und
der Anteil an ungesättigten C₁₈-Acylgruppen, vorzugsweise an Acylgruppen abgeleitet von Ölsäure, 4,0 - 10,0 Gew.-% ist,
und wobei die genannten Anteile auf die Gesamtmenge der in dem einen oder den mehreren Acylglycinaten enthaltenen Acylgruppen bezogen sind, aber nicht auf Basis der Acylgruppen selbst, sondern auf Basis der zu den Acylgruppen korrespondierenden Fettsäurechloride berechnet werden.

Die Acylgruppen in der einen oder den mehreren Acylglycinaten der Formel (I) sind vorzugsweise linear.

In dem einen oder den mehreren Acylglycinaten der Formel (I) sind die Acylgruppen mit 12, 14 und 16 Kohlenstoffatomen vorzugsweise gesättigt.

Vorzugsweise ist in dem einen oder den mehreren Acylglycinaten der Formel (I) der Anteil an Acylgruppen mit 8 bis 18 Kohlenstoffatomen 95 Gew.-% oder größer und besonders bevorzugt 96,5 Gew.-% oder größer, wobei die genannten Anteile auf die Gesamtmenge der in dem einen oder den mehreren Acylglycinaten enthaltenen Acylgruppen bezogen sind, aber nicht auf Basis der Acylgruppen selbst, sondern auf Basis der zu den Acylgruppen korrespondierenden Fettsäurechloride berechnet werden.

Weiterhin bevorzugt sind Zusammensetzungen enthaltend Natriumcocoylglycinat, wobei der Cocoylschnitt einer natürlichen Kokosfettsäure entspricht.

Q⁺C^{l-} wird im Rahmen der vorliegenden Anmeldung auch als QCI bezeichnet, z. B. Na⁺Cl⁻ als NaCl.

In den Verbindungen der Formeln (I) und (II) sowie in Q⁺Cl⁻ ist Q⁺ ausgewählt aus Na⁺ und K⁺ und vorzugsweise steht Q⁺ für Na⁺.

Die erfindungsgemäßen Zusammensetzungen enthalten die eine oder die mehreren Substanzten Q⁺Cl⁻ in Mengen größer oder gleich 1,0 Gew.-%, vorzugsweise in Mengen von 1,0 bis 8,0 Gew.-%, besonders bevorzugt in Mengen von 2,0 bis 7,0 Gew.-% und insbesondere bevorzugt in Mengen von 4,0 bis 6,0 Gew.-%, bezogen auf die gesamte Zusammensetzung. Dies ist z.B. für kosmetische Anwendungen sehr vorteilhaft, weil eine gesonderte Zugabe von Q⁺Cl⁻ als Viskositätsregulierer in den mit Hilfe von erfindungsgemäßen Zusammensetzungen hergestellten kosmetischen Endformulierungen entfallen kann.

Die erfindungsgemäßen Zusammensetzungen können somit beispielsweise vorteilhaft für kosmetische Anwendungen und zur Herstellung kosmetischer Zubereitungen verwendet werden.

Die erfindungsgemäßen Zusammensetzungen enthalten das eine oder die mehreren Fettsäuresalze der Formel (II) in Mengen kleiner oder gleich 3,0 Gew.-%, vorzugsweise in Mengen von 0,01 bis 2,0 Gew.-%, besonders bevorzugt in Mengen von 0,1 bis 2,0 Gew.-% und insbesondere bevorzugt in Mengen von 0,1 bis 1,5 Gew.-%, bezogen auf die gesamte Zusammensetzung.

Ein weiterer Vorteil der erfindungsgemäßen Zusammensetzungen ist, dass diese nur geringe Mengen an Fettsäuresalz der Formel (II) und dadurch Acylglycinate der Formel (I) mit hoher Reinheit enthalten.

Vorzugsweise ist die Reinheit der in den erfindungsgemäßen Zusammensetzungen enthaltenen Acylglycinate der Formel (I) und vorzugsweise der Natriumcocoylglycinate 90 % oder größer und vorzugsweise 92 % oder größer. Diese Reinheit ist auf die Summe an Fettsäuresalz der Formel (II) und Acylglycinat der Formel (I) bezogen. Sie berechnet sich nach der Formel "Reinheit der Acylglycinate = [Menge an Acylglycinat : (Menge an Acylglycinat + Menge an Fettsäuresalz)]".

Die erfindungsgemäßen Zusammensetzungen sind bei 40 °C flüssig und einphasig.

Sehr vorteilhaft ist auch die relativ geringe Viskosität und damit eine einfache Handhabung der erfindungsgemäßen Zusammensetzungen.

Vorzugsweise weisen die erfindungsgemäßen Zusammensetzungen Viskositäten bei 40 °C von kleiner als 5 000 mPa·s, besonders bevorzugt von 50 bis 2 000 mPa·s und insbesondere bevorzugt von 80 bis 500 mPa·s auf.

Bei dem für die Bestimmung der Viskosität verwendeten Gerät handelt es sich um ein Rotameter der Firma Thermo Haake (Viskotester 550). Als Spindel wurde MV-DIN (45,3 Umdrehungen / Minute) verwendet.

Die erfindungsgemäßen Zusammensetzungen enthalten keine organischen Lösungsmittel, wie beispielsweise niedere Alkohole, Diole oder andere organische Lösungsmittel.

In einer besonders bevorzugten Ausführungsform der Erfindung bestehen die erfindungsgemäßen Zusammensetzungen aus den Komponenten a), b), c) und d).

In einer weiteren besonders bevorzugten Ausführungsform der Erfindung bestehen die erfindungsgemäßen Zusammensetzungen aus den Komponenten a), b), c), d) und H₂NCH₂COO⁻ Q⁺, wobei Q⁺ für ein Kation ausgewählt aus Na⁺ und K⁺ und vorzugsweise für Na⁺ steht. In diesen erfindungsgemäßen Zusammensetzungen ist die Verbindung H₂NCH₂COO⁻ Q⁺, bezogen auf die gesamte erfindungsgemäße Zusammensetzung, vorzugsweise von 0,01 bis 2,0 Gew.-% und besonders bevorzugt von 0,05 bis 1,5 Gew.-%, enthalten.

Ein weiterer Gegenstand der Erfindung betrifft auch die Herstellung der erfindungsgemäßen Zusammensetzungen durch das erfindungsgemäße Verfahren.

Wie bereits erwähnt sind die erfindungsgemäßen Zusammensetzungen in vorteilhafter Weise zur Herstellung kosmetischer Zubereitungen geeignet.

Ein weiterer Gegenstand der Erfindung betrifft daher die Verwendung der erfindungsgemäßen Zusammensetzungen zur Herstellung einer kosmetischen Zubereitung. Besonders vorteilhaft dabei ist, dass die erfindungsgemäßen Zusammensetzungen wie aus dem erfindungsgemäßen Verfahren erhalten, d. h. ohne weitere Aufarbeitung oder Aufreinigung, verwendet werden können.

Die erfindungsgemäßen Zusammensetzungen sind zudem in vorteilhafter Weise als Tenside in kosmetischen Zubereitungen geeignet.

Ein weiterer Gegenstand der Erfindung betrifft daher die Verwendung der erfindungsgemäßen Zusammensetzungen als Tenside in kosmetischen Zubereitungen. Die erfindungsgemäßen Zusammensetzungen können auch hierbei direkt wie aus dem erfindungsgemäßen Verfahren erhalten verwendet werden.

Die nachfolgenden Beispiele und Anwendungen sollen die Erfindung näher erläutern, ohne sie jedoch darauf zu beschränken. Bei allen Prozentangaben handelt es sich um Gewichtsprozent (Gew.-%), sofern nicht explizit anders angegeben.

### Vergleichsbeispiel 1

### Cocoylchlorid (A): Cocosschnitt mit reduziertem Anteil an C₁₆ und C₁₈

Spezifikation des verwendeten Cocoylchlorids:

| | |
|---|---|
| C₈/C₁₀ gesättigt: | 10,0 - 14,0 Gew.-% |
| C₁₂: | 60,0 - 62,0 Gew.-% |
| C₁₄: | 19,0 - 24,0 Gew.-% |
| C₁₆: | 3,0 - 10,0 Gew.-% |
| C₁₈ gesättigt: | < 2,0 Gew.-% |

37,8 g (0,504 mol) Glycin wird in 276 g VE-Wasser (vollentsalztes Wasser) unter Rühren gelöst und der pH-Wert (telquel) mit Natronlauge (33 Gew.-% in Wasser) auf 12 - 13 eingestellt. Anschließend wird unter Rühren auf 30 - 35 °C aufgeheizt und 106,4 g (0,478 mol) Cocoylchlorid (A) innerhalb 6 Stunden bei 30 - 35 °C unter Kühlung des Reaktionsgemisches zudosiert. Der pH-Wert wird durch gleichzeitige Dosierung von Natronlauge (33 Gew.-% in Wasser) bei 12 - 13 gehalten. Gegen Ende der Dosierung des Cocoylchlorids lässt man den pH-Wert auf 9,5 - 10,5 abfallen. Zur Vervollständigung der Reaktion wird noch 2 Stunden bei pH 9,5 - 10,5 gerührt.

Das erhaltene Produkt hat folgende Eigenschaften:

| | |
|---|---|
| Bei 25 °C: | flüssig, opal |
| Trockenrückstand (1 Stunde, 140 °C): | 31,0 Gew.-% |
| Glycinsalz (HPLC): | 0,6 Gew.-% |
| Fettsäuresalz (HPLC): | 0,6 Gew.-% |
| NaCl (Titration): | 5,3 Gew.-% |
| Aktivgehalt: | 24,5 Gew.-% |
| Viskosität (35 °C): | 756 mPa·s |

Die Berechnung der Gewichtsmenge an Acylglycinat in den Zusammensetzungen erfolgt durch die Formel "Gewichtsmenge an Acylglycinat = Trockenrückstand - Fettsäuresalz - Glycinsalz - Q⁺Cl⁻". Der Wert wird im Rahmen der vorliegenden Anmeldung als "Aktivgehalt" bezeichnet.

Vergleichbeispiel 1 entspricht dem Stand der Technik der WO 2009/065530. Hierbei wird eine bei Raumtemperatur (25 °C) flüssige Lösung von Natriumcocoylglycinat erhalten, die jedoch auf einem kommerziell weniger verfügbaren Kettenschnitt beruht.

### Vergleichsbeispiel 2:

### Cocosschnitt mit erhöhtem Anteil an C_{16/18}, hydriert, aber ohne Anteil an C₈ und C₁₀

Verteilung des verwendeten Cocoylchlorids (B) :

| | |
|---|---|
| C₁₂: | 55,6 Gew.-% |
| C₁₄: | 23,0 Gew.-% |
| C₁₆: | 11,1 Gew.-% |
| C₁₈ gesättigt: | 10,3 Gew.-% |

36,0 g (0,480 mol) Glycin wird in 280 g VE-Wasser unter Rühren gelöst und der pH-Wert (telquel) mit Natronlauge (33 Gew.-% in Wasser) auf 12-13 eingestellt. Anschließend wird unter Rühren auf 30 - 35 °C aufgeheizt und 109,6 g (0,456 mol) Cocoylchlorid (B) innerhalb 6 Stunden bei 30 - 35 °C unter Kühlung des Reaktionsgemisches zudosiert. Der pH-Wert wird durch gleichzeitige Dosierung von Natronlauge (33 Gew.-% in Wasser) bei 12 - 13 gehalten. Im Laufe der Dosierung des Cocoylchlorids wird der Ansatz immer zähflüssiger bis er nicht mehr rührbar ist. Durch Zugabe von 110 g Wasser und anschließende Erhöhung der Reaktionstemperatur auf 40 °C bleibt der Ansatz einigermaßen rührbar. Gegen Ende der Dosierung des Cocoylchlorids lässt man den den pH-Wert auf 9,5 - 10,5 abfallen. Zur Vervollständigung der Reaktion wird noch 2 Stunden bei pH 9,5 - 10,5 gerührt.

Das erhaltene Produkt hat folgende Eigenschaften:

| | |
|---|---|
| Bei 25 °C: | flüssig, trüb |
| Trockenrückstand (1 Stunde, 140 °C): | 25,8 Gew.-% |
| Glycinsalz (HPLC): | 0,8 Gew.-% |
| Fettsäuresalz (HPLC): | 1,2 Gew.-% |
| NaCl (Titration): | 4,1 Gew.-% |
| Aktivgehalt: | 19,7 Gew.-% |
| Viskosität: | nicht bestimmt |

Vergleichsbeispiel 2 zeigt, dass höhere Gehalte an gesättigten C_{16/18}-Fettsäurechloriden zu niedrigkonzentrierten, nicht handhabbaren Glycinatlösungen führen, sofern im Säurechlorid keine Anteile an C₈-, C₁₀- und ungesättigtem C₁₈-Säurechlorid vorliegen.

### Beispiel 1:

### Cocosschnitt mit Anteilen an C₈ und C₁₀ und ungesättigten Anteilen an C₁₈

**Verteilung des verwendeten Cocoylchlorids:**

| Fettsäurechlorid | Gew.-% |
|---|---|
| C₆-Fettsäurechlorid | 0,5 |
| C₈-Fettsäurechlorid, gesättigt | 7,4 |
| C₁₀-Fettsäurechlorid, gesättigt | 5,9 |
| C₁₂-Fettsäurechlorid | 49,4 |
| C₁₄-Fettsäurechlorid | 18,5 |
| C₁₆-Fettsäurechlorid | 8,4 |
| C₁₈-Fettsäurechlorid, ungesättigt | 5,2 |
| C₁₈-Fettsäurechlorid, gesättigt | 2,2 |
| Summe x | 2,5 |

| | |
|---|---|
| x: unbestimmte Substanzen | |

18,9 g (0,252 mol) Glycin wird in 146 g VE-Wasser unter Rühren gelöst und der pH-Wert (telquel) mit Natronlauge (33 Gew.-% in Wasser) auf 12 - 13 eingestellt. Anschließend wird unter Rühren auf 35 - 40°C aufgeheizt und 53,1 g (0,239 mol) Cocoylchlorid innerhalb 6 Stunden bei 35 - 40°C unter Kühlung des Reaktionsgemisches zudosiert. Der pH-Wert wird durch gleichzeitige Dosierung von Natronlauge (33 Gew.-% in Wasser) bei 12 - 13 gehalten. Gegen Ende der Dosierung des Cocoylchlorids lässt man den pH-Wert auf 9,5 - 10,5 abfallen. Zur Vervollständigung der Reaktion wird noch 2 Stunden bei 40 °C und pH 9,5 - 10,5 gerührt.

Das erhaltene Produkt hat folgende Eigenschaften:

| | |
|---|---|
| Bei 40 °C: | dünnflüssig, klar, homogen |
| Trockenrückstand (1 Stunde, 140 °C): | 30,0 Gew.-% |
| Glycinsalz (HPLC): | 0,9 Gew.-% |
| Fettsäuresalz (HPLC): | 1,8 Gew.-% |
| NaCl (Titration): | 5,0 Gew.-% |
| Aktivgehalt: | 22,3 Gew.-% |
| Viskosität (40 °C): | 100 mPa·s |

Das erfindungsgemäße Beispiel 1 zeigt, dass mit Fettsäurechloriden, die sowohl signifikante Mengen C₈- und C₁₀-Fettsäurechlorid, als auch ungesättigtes C₁₈-Fettsäurechlorid enthalten (Kettenschnitt entsprechend einem natürlich vorkommenden Kokosfettsäureschnitt), bei 40 °C homogene Glycinatlösungen mit einem hohen Aktivgehalt erhalten werden.

### Vergleichsbeispiel 3:

### Cocosschnitt mit ungesättigten Anteilen an C₁₈, aber geringen Anteilen an C₈ und C₁₀

**Verteilung des verwendeten Cocoylchlorids:**

| Fettsäurechlorid | Gew.-% |
|---|---|
| C₈-Fettsäurechlorid, gesättigt | 0,1 |
| C₁₀-Fettsäurechlorid, gesättigt | 0,5 |
| C₁₂-Fettsäurechlorid | 55,1 |
| C₁₄-Fettsäurechlorid | 23,0 |
| C₁₆-Fettsäurechlorid | 10,7 |
| C₁₈-Säurechlorid, ungesättigt | 6,8 |
| C₁₈-Säurechlorid, gesättigt | 2,9 |
| Summe x | 1,0 |

| | |
|---|---|
| x: unbestimmte Substanzen | |

18,9 g (0,252 mol) Glycin wird in 155 g VE-Wasser unter Rühren gelöst und der pH-Wert (telquel) mit Natronlauge (33 Gew.-% in Wasser) auf 12 - 13 eingestellt. Anschließend wird unter Rühren auf 35 - 40 °C aufgeheizt und 57,2 g (0,239 mol) Cocoylchlorid innerhalb 6 Stunden bei 35 - 40 °C unter Kühlung des Reaktionsgemisches zudosiert. Der pH-Wert wird durch gleichzeitige Dosierung von Natronlauge (33 Gew.-% in Wasser) bei 12 - 13 gehalten. Im Laufe der Dosierung des Cocoylchlorids wird der Ansatz immer zähflüssiger bis er schließlich nicht mehr rührbar ist. Dies macht es notwendig, die Temperatur zuerst auf 45 °C und dann auf 50 °C anzuheben. Trotzdem bleibt der Ansatz viskos. Gegen Ende der Dosierung des Cocoylchlorids lässt man den pH-Wert auf 9,5 - 10,5 abfallen. Zur Vervollständigung der Reaktion wird noch 2 Stunden bei 50 °C und pH 9,5 - 10,5 gerührt.

Das erhaltene Produkt hat folgende Eigenschaften:

| | |
|---|---|
| Bei 40 °C: | nicht rührbare zähe Masse |
| Trockenrückstand (1 Stunde, 140 °C): | 31,2 Gew.-% |
| Glycinsalz (HPLC): | 2,5 Gew.-% |
| Fettsäuresalz (HPLC): | 4,4 Gew.-% |
| NaCl (Titration): | 4,6 Gew.-% |
| Aktivgehalt: | 19,7 Gew.-% |
| Viskosität: | nicht bestimmt |

Das Vergleichsbeispiel 3 zeigt, dass mit Fettsäurechloriden, die keine signifikanten Mengen C₈- und C₁₀-Fettsäurechlorid, aber andererseits signifikante Mengen ungesättigtes C₁₈-Fettsäurechlorid enthalten (entsprechend einem kommerziellen, natürlich vorkommenden Kokosfettsäureschnitt, der gekappt wurde), als Produkte bei 40 °C lediglich nicht rührbare, zähe Massen mit geringer Reinheit erhalten werden.

### Beispiel 2:

### Cocosschnitt mit ungesättigten Anteilen aus Vergleichsbeispiel 3, aufgestockt mit C₈ und C₁₀

**Verteilung des verwendeten Cocoylchlorids:**

| Fettsäurechlorid | Gew.-% |
|---|---|
| C₈-Fettsäurechlorid, gesättigt | 5,0 |
| C₁₀-Fettsäurechlorid, gesättigt | 8,0 |
| C₁₂-Fettsäurechlorid | 48,3 |
| C₁₄-Fettsäurechlorid | 20,1 |
| C₁₆-Fettsäurechlorid | 9,3 |
| C₁₈-Fettsäurechlorid, ungesättigt | 5,8 |
| C₁₈-Fettsäurechlorid, gesättigt | 2,5 |
| Summe x | 1,0 |

| | |
|---|---|
| x: unbestimmte Substanzen | |

18,9 g (0,252 mol) Glycin wird in 156 g VE-Wasser unter Rühren gelöst und der pH-Wert (telquel) mit Natronlauge (33 Gew.-% in Wasser) auf 12 - 13 eingestellt.

Anschließend wird unter Rühren auf 35 - 40 °C aufgeheizt und 55,5 g (0,239 mol) Cocoylchlorid innerhalb 6 Stunden bei 35 - 40 °C unter Kühlung des Reaktionsgemisches zudosiert. Der pH-Wert wird durch gleichzeitige Dosierung von Natronlauge (33 Gew.-% in Wasser) bei 12 - 13 gehalten. Der Ansatz ist gut rührbar. Gegen Ende der Dosierung des Cocoylchlorids lässt man den pH-Wert auf 9,5 - 10,5 abfallen. Zur Vervollständigung der Reaktion wird noch 2 Stunden bei 40 °C und pH 9,5 - 10,5 gerührt.

Das erhaltene Produkt hat folgende Eigenschaften:

| | |
|---|---|
| Bei 40 °C: | dünnflüssig, klar, homogen |
| Trockenrückstand (1 Stunde, 140 °C): | 29,7 Gew.-% |
| Glycinsalz (HPLC): | 0,8 Gew.-% |
| Fettsäuresalz (HPLC): | 1,9 Gew.-% |
| NaCl (Titration): | 4,6 Gew.-% |
| Aktivgehalt: | 22,4 Gew.-% |
| Viskosität (40 °C): | 388 mPa·s |

Das erfindungsgemäße Beispiel 2 zeigt, dass mit Fettsäurechloriden, die sowohl signifikante Mengen an C₈- und C₁₀-Fettsäurechlorid, als auch an ungesättigtem C₁₈-Fettsäurechlorid enthalten (Kettenschnitt entsprechend einem natürlich vorkommenden Kokosfettsäureschnitt), bei 40 °C homogene Glycinatlösungen mit hohem Aktivgehalt erhalten werden.

### Vergleichsbeispiel A:

### Schaumvermögen der erfindungsgemäßen Zusammensetzungen:

Das Schaumvermögen wurde in einem SITA Foamtester bei einer Tensidkonzentration von 0,01 Gew.-% bei pH = 9 und 37 °C gemessen. In der folgenden Tabelle 1 sind die Schaumhöhen in Abhängigkeit den Rührzyklen wiedergegeben.

**Tabelle 1: gemessene Schaumhöhen [mm] in Abhängigkeit von den Rührzyklen**

| | | | | | | | | | |
|---|---|---|---|---|---|---|---|---|---|
| Rührzyklen | 2 | 4 | 6 | 8 | 10 | 15 | 20 | 25 | 30 |
| Schaumhöhen [mm] Beispiel 1 | 22 | 60 | 98 | 130 | 150 | 195 | 225 | 235 | 245 |
| Schaumhöhen [mm] Vergleichsbeispiel 1 | 96 | 130 | 130 | 130 | 130 | 130 | 130 | 130 | 130 |

Die in Tabelle 1 wiedergegebenen Versuchsergebnisse zeigen, dass das erfindungsgemäße Acylglycinat bzw. die erfindungsgemäßen Zusammensetzungen deutlich höhere erzielbare Gesamtschaumhöhen als das Acylglycinat bzw. die Zusammensetzung aus Vergleichsbeispiel 1 ermöglichen. Die hohen erzielbaren Gesamtschaumhöhen führen z. B. in kosmetischen Formulierungen zu cremigen Schäumen, die sensorisch ansprechend sind.

## Patentansprüche

1. Verfahren zur Herstellung von Acylglycinaten der Formel (I) 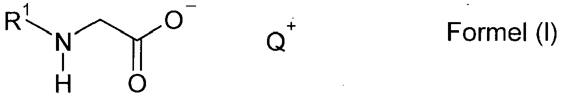 worin
R¹ für eine lineare oder verzweigte, gesättigte Alkanoylgruppe mit 6 bis 30 Kohlenstoffatomen oder für eine lineare oder verzweigte, ein- oder mehrfach ungesättigte Alkenoylgruppe mit 6 bis 30 Kohlenstoffatomen steht, und
Q⁺ für ein Kation ausgewählt aus den Alkalimetallkationen Na⁺ und K⁺ steht,
**dadurch gekennzeichnet, dass** Glycin mit Fettsäurechlorid R¹Cl, wobei R¹ die in Formel (I) angegebene Bedeutung besitzt,
und für eine Acylgruppe mit 6 bis 30 Kohlenstoffatomen steht, wobei der Anteil an Fettsäurechloriden mit gesättigten C₈-Acylgruppen 5,0 - 8,0 Gew.-% ist,
der Anteil an Fettsäurechloriden mit gesättigten C₁₀-Acylgruppen 5,0 - 8,0 Gew.-% ist,
der Anteil an Fettsäurechloriden mit C₁₂-Acylgruppen 44,0 - 50,0 Gew.-% ist,
der Anteil an Fettsäurechloriden mit C₁₄-Acylgruppen 14,0 - 20,0 Gew.-% ist,
der Anteil an Fettsäurechloriden mit C₁₆-Acylgruppen 8,0 - 10,0 Gew.-% ist,
der Anteil an Fettsäurechloriden mit gesättigten C₁₈-Acylgruppen 1,0 - 3,0 Gew.-% ist und
der Anteil an Fettsäurechloriden mit ungesättigten C₁₈-Acylgruppen 4,0 - 10,0 Gew.-% ist,
jeweils bezogen auf die Gesamtmenge an eingesetztem Fettsäurechlorid, in Wasser und in Gegenwart einer basischen Alkaliverbindung, die Kationen Q⁺ ausgewählt aus Na⁺ und K⁺ liefert, aber in Abwesenheit von organischen Lösungsmitteln, bei 25 - 50 °C umgesetzt wird.

2. Verfahren nach Anspruch 1, **dadurch gekennzeichnet, dass** als basische Alkaliverbindungen Carbonate oder Hydroxide der Alkalimetallkationen Na⁺ oder K⁺ oder Mischungen davon eingesetzt werden.

3. Verfahren nach Anspruch 1 oder 2, **dadurch gekennzeichnet, dass** es bei einem pH-Wert von 9 bis 13 durchgeführt wird.

4. Verfahren nach einem oder mehreren der Ansprüche 1 bis 3, **dadurch gekennzeichnet, dass** das molare Verhältnis von Glycin zu Fettsäurechlorid R¹Cl von 1,1 : 1,0 bis 1,0 : 1,0 ist.

5. Zusammensetzung enthaltend
a) ein oder mehrere Acylglycinate der Formel (I) 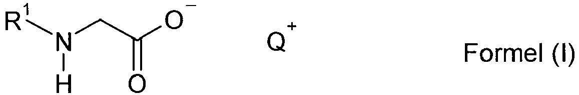 worin
R¹ für eine lineare oder verzweigte, gesättigte Alkanoylgruppe mit 6 bis 30 Kohlenstoffatomen oder für eine lineare oder verzweigte, ein- oder mehrfach ungesättigte Alkenoylgruppe mit 6 bis 30 Kohlenstoffatomen steht, und
Q⁺ für ein Kation ausgewählt aus den Alkalimetallkationen Na⁺ und K⁺ steht,
in Mengen von 21,0 - 28,0 Gew.-%, bezogen auf die gesamte Zusammensetzung, und wobei in dem einen oder den mehreren Acylglycinaten der Formel (I)
der Anteil an gesättigten C₈-Acylgruppen 5,0 - 8,0 Gew.-% ist,
der Anteil an gesättigten C₁₀-Acylgruppen 5,0 - 8,0 Gew.-% ist,
der Anteil an C₁₂-Acylgruppen 44,0 - 50,0 Gew.-% ist,
der Anteil an C₁₄-Acylgruppen 14,0 - 20,0 Gew.-% ist,
der Anteil an C₁₆-Acylgruppen 8,0 - 10,0 Gew.-% ist,
der Anteil an gesättigten C₁₈-Acylgruppen 1,0 - 3,0 Gew.-% ist und
der Anteil an ungesättigten C₁₈-Acylgruppen 4,0 - 10,0 Gew.-% ist,
und wobei die genannten Anteile auf die Gesamtmenge der in dem einen oder den mehreren Acylglycinaten enthaltenen Acylgruppen bezogen sind, aber nicht auf Basis der Acylgruppen selbst, sondern auf Basis der zu den Acylgruppen korrespondierenden Fettsäurechloride berechnet werden,
b) ein oder mehrere Substanzen Q⁺Cl⁻, worin Q⁺ die Bedeutung von Q⁺ aus Formel (I) hat, in Mengen größer oder gleich 1,0 Gew.-%, bezogen auf die gesamte Zusammensetzung,
c) ein oder mehrere Fettsäuresalze der Formel (II) 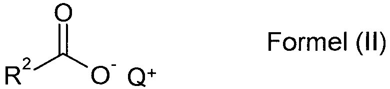 worin
R²CO die Bedeutung von R¹ aus Formel (I) und
Q⁺ die Bedeutung von Q⁺ aus Formel (I) hat,
in Mengen kleiner oder gleich 3,0 Gew.-%, bezogen auf die gesamte Zusammensetzung, und
d) Wasser,
e) aber keine organischen Lösungsmittel.

6. Zusammensetzung nach Anspruch 5, **dadurch gekennzeichnet, dass** sie die Komponente a) in Mengen von 23,0 - 27,0 Gew.-%, bezogen auf die gesamte Zusammensetzung, enthält.

7. Zusammensetzung nach Anspruch 5 oder 6, **dadurch gekennzeichnet, dass** Q⁺ für Na⁺ steht.

8. Zusammensetzung nach einem oder mehreren der Ansprüche 5 bis 7, **dadurch gekennzeichnet, dass** sie die eine oder die mehreren Substanzen Q⁺Cl⁻ in Mengen von 1,0 bis 8,0 Gew.-%, bezogen auf die gesamte Zusammensetzung, enthält.

9. Zusammensetzung nach einem oder mehreren der Ansprüche 5 bis 8, **dadurch gekennzeichnet, dass** sie das eine oder die mehreren Fettsäuresalze der Formel (II) in Mengen von 0,01 bis 2,0 Gew.-%, bezogen auf die gesamte Zusammensetzung, enthält.

10. Zusammensetzung nach einem oder mehreren der Ansprüche 5 bis 9, **dadurch gekennzeichnet, dass** die Reinheit der darin enthaltenen Acylglycinate der Formel (I) 90 % oder größer ist, wobei diese Reinheit auf die Summe an Fettsäuresalz der Formel (II) und Acylglycinat der Formel (I) bezogen ist und sich nach der Formel "Reinheit der Acylglycinate = [Menge an Acylglycinat : (Menge an Acylglycinat + Menge an Fettsäuresalz)]" berechnet.

11. Zusammensetzung nach einem oder mehreren der Ansprüche 5 bis 10, **dadurch gekennzeichnet, dass** sie eine Viskosität bei 40 °C von kleiner als 5 000 mPa·s aufweist.

12. Verwendung einer Zusammensetzung nach einem oder mehreren der Ansprüche 5 bis 11 zur Herstellung einer kosmetischen Zubereitung.

13. Verwendung einer Zusammensetzung nach einem oder mehreren der Ansprüche 5 bis 11 als Tensid in einer kosmetischen Zubereitung.

## Claims

1. A process for preparing acylglycinates of the formula (I) 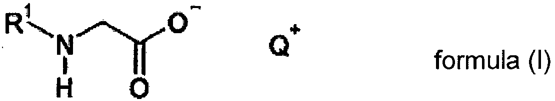 in which
R¹ is a linear or branched, saturated alkanoyl group having 6 to 30 carbon atoms, or a linear or branched, mono- or polyunsaturated alkenoyl group having 6 to 30 carbon atoms, and
Q⁺ is a cation selected from the alkali metal cations Na⁺ and K⁺,
wherein glycine is reacted with fatty acid chloride R¹Cl where R¹ is as defined in formula (I),
and is an acyl group having 6 to 30 carbon atoms, where the proportion of fatty acid chlorides having saturated C₈ acyl groups is 5.0-8.0% by weight,
the proportion of fatty acid chlorides having saturated C₁₀ acyl groups is 5.0-8.0% by weight,
the proportion of fatty acid chlorides having C₁₂ acyl groups is 44.0-50.0% by weight,
the proportion of fatty acid chlorides having C₁₄ acyl groups is 14.0-20.0% by weight,
the proportion of fatty acid chlorides having C₁₆ acyl groups is 8.0-10.0% by weight,
the proportion of fatty acid chlorides having saturated C₁₈ acyl groups is 1.0-3.0% by weight, and
the proportion of fatty acid chlorides having unsaturated C₁₈ acyl groups is 4.0-10.0% by weight,
based in each case on the total amount of fatty acid chloride used, in water and in the presence of a basic alkali metal compound which provides cations Q⁺ selected from Na⁺ and K⁺, but in the absence of organic solvents, at 25-50°C.

2. The process as claimed in claim 1, wherein the basic alkali metal compounds used are carbonates or hydroxides of the alkali metal cations Na⁺ or K⁺ or mixtures thereof.

3. The process as claimed in claim 1 or 2, which is performed at a pH of 9 to 13.

4. The process as claimed in one or more of claims 1 to 3, wherein the molar ratio of glycine to fatty acid chloride R¹Cl is from 1.1:1.0 to 1.0:1.0.

5. A composition comprising
a) one or more acylglycinates of the formula (I) 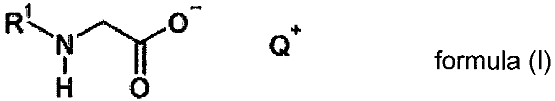 in which
R¹ is a linear or branched, saturated alkanoyl group having 6 to 30 carbon atoms, or a linear or branched, mono- or polyunsaturated alkenoyl group having 6 to 30 carbon atoms, and
Q⁺ is a cation selected from the alkali metal cations Na⁺ and K⁺,
in amounts of 21.0-28.0% by weight, based on the overall composition, and where, in the one or more acylglycinates of the formula (I),
the proportion of saturated C₈ acyl groups is 5.0-8.0% by weight,
the proportion of saturated C₁₀ acyl groups is 5.0-8.0% by weight,
the proportion of C₁₂ acyl groups is 44.0-50.0% by weight,
the proportion of C₁₄ acyl groups is 14.0-20.0% by weight,
the proportion of C₁₆ acyl groups is 8.0-10.0% by weight,
the proportion of saturated C₁₈ acyl groups is 1.0-3.0% by weight and
the proportion of unsaturated C₁₈ acyl groups is 4.0-10.0% by weight,
and where the proportions mentioned are based on the total amount of acyl groups present in the one or more acylglycinates, but are calculated not on the basis of the acyl groups themselves but on the basis of the fatty acid chlorides corresponding to the acyl groups,
b) one or more substances Q⁺Cl⁻ in which Q⁺ is as defined for Q⁺ in formula (I), in amounts greater than or equal to 1.0% by weight, based on the overall composition,
c) one or more fatty acid salts of the formula (II) 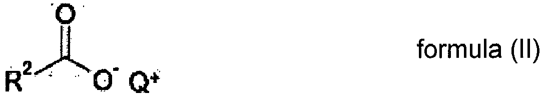 in which
R²CO is as defined for R¹ in formula (I) and
Q⁺ is as defined for Q⁺ in formula (I),
in amounts less than or equal to 3.0% by weight, based on the overall composition, and
d) water,
e) but no organic solvents.

6. The composition as claimed in claim 5, which comprises component a) in amounts of 23.0-27.0% by weight, based on the overall composition.

7. The composition as claimed in claim 5 or 6, wherein Q⁺ is Na⁺.

8. The composition as claimed in one or more of claims 5 to 7, which comprises the one or more substances Q⁺Cl⁻ in amounts of 1.0 to 8.0% by weight, based on the overall composition.

9. The composition as claimed in one or more of claims 5 to 8, which comprises the one or more fatty acid salts of the formula (II) in amounts of 0.01 to 2.0% by weight, based on the overall composition.

10. The composition as claimed in one or more of claims 5 to 9, wherein the purity of the acylglycinates of the formula (I) present therein is 90% or greater, this purity being based on the sum total of fatty acid salt of the formula (II) and acylglycinate of the formula (I), and being calculated by the formula "purity of the acylglycinates = [amount of acylglycinate:(amount of acylglycinate + amount of fatty acid salt)]".

11. The composition as claimed in one or more of claims 5 to 10, which has a viscosity at 40°C of less than 5000 mPa·s.

12. The use of a composition as claimed in one or more of claims 5 to 11 for production of a cosmetic formulation.

13. The use of a composition as claimed in one or more of claims 5 to 11 as a surfactant in a cosmetic formulation.

## Revendications

1. Procédé de préparation d'acylglycinates de formule (I) 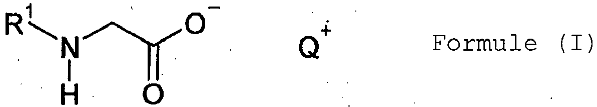 dans laquelle
R¹ représente un groupe alcanoyle saturé à chaîne droite ou ramifiée ayant 6 à 30 atomes de carbone ou un groupe alcénoyle mono- ou polyinsaturé à chaîne droite ou ramifiée ayant 6 à 30 atomes de carbone, et
Q⁺ est un cation choisi parmi les cations de métaux alcalins Na⁺ et K⁺,
**caractérisé en ce qu'**on fait réagir à 25 à 50°C de la glycine avec un chlorure d'acide gras R¹Cl, où R¹ a les significations données dans la formule (I),
et représente un groupe acyle ayant 6 à 30 atomes de carbone,
la proportion des chlorures d'acides gras ayant des groupes acyle saturés en C₈ est de 5,0 à 8,0 % en poids,
la proportion des chlorures d'acides gras ayant des groupes acyle saturés en C₁₀ est de 5,0 à 8,0 % en poids,
la proportion des chlorures d'acides gras ayant des groupes acyle en C₁₂ est de 44,0 à 50,0 % en poids,
la proportion des chlorures d'acides gras ayant des groupes acyle en C₁₄ est de 14,0 à 20,0 % en poids,
la proportion des chlorures d'acides gras ayant des groupes acyle en C₁₆ est de 8,0 à 10,0 % en poids,
la proportion des chlorures d'acides gras ayant des groupes acyle saturés en C₁₈ est de 1,0 à 3,0 % en poids, et
la proportion des chlorures d'acides gras ayant des groupes acyle insaturés en C₁₈ est de 4,0 à 10,0 % en poids,
chacune par rapport à la quantité totale du chlorure d'acide gras utilisé,
dans de l'eau et en présence d'un composé alcalin basique, qui fournit des cations Q⁺ choisis parmi Na⁺ et K⁺, mais en l'absence de solvants organiques.

2. Procédé selon la revendication 1, **caractérisé en ce qu'**on utilise en tant que composés alcalins basiques des carbonates ou des hydroxydes des cations de métaux alcalins Na⁺ ou K⁺ ou des mélanges de ceux-ci.

3. Procédé selon la revendication 1 ou 2, **caractérisé en ce qu'**il est mis en oeuvre à un pH de 9 à 13.

4. Procédé selon l'une ou plusieurs des revendications 1 à 3, **caractérisé en ce que** le rapport en moles de la glycine au chlorure d'acide gras R¹Cl est de 1,1:1,0 à 1,0:1,0.

5. Composition contenant
a) un ou plusieurs acylglycinates de formule (I) 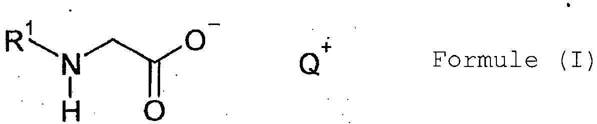 dans laquelle
R¹ représente un groupe alcanoyle saturé à chaîne droite ou ramifiée ayant 6 à 30 atomes de carbone ou un groupe alcénoyle mono- ou polyinsaturé à chaîne droite ou ramifiée ayant 6 à 30 atomes de carbone, et
Q⁺ est un cation choisi parmi les cations de métaux alcalins Na⁺ et K⁺,
en des quantités de 21,0 à 28,0 % en poids par rapport à la composition totale,
et dans laquelle, dans le ou les acylglycinates de formule (I),
la proportion des groupes acyle saturés en C₈ est de 5,0 à 8,0 % en poids,
la proportion des groupes acyle saturés en C₁₀ est de 5,0 à 8,0 % en poids,
la proportion des groupes acyle en C₁₂ est de 44,0 à 50,0 % en poids,
la proportion des groupes acyle en C₁₄ est de 14, 0 à 20,0 % en poids,
la proportion des groupes acyle en C₁₆ est de 8,0 à 10,0 % en poids,
la proportion des groupes acyle saturés en C₁₈ est de 1,0 à 3,0 % en poids, et
la proportion des groupes acyle insaturés en C₁₈ est de 4,0 à 10,0 % en poids,
et dans laquelle les proportions mentionnées sont rapportées à la quantité totale des groupes acyle contenus dans le ou les acylglycinates, mais sont calculées non pas sur la base des groupes acyle proprement dits, mais sur la base des chlorures d'acides gras correspondant aux groupes acyle,
b) une ou plusieurs substances Q⁺Cl⁻, où Q⁺ a les significations de Q⁺ de la formule (I), en des quantités supérieures ou égales à 1,0 % en poids par rapport à la composition totale,
c) un ou plusieurs sels d'acides gras de formule (II) 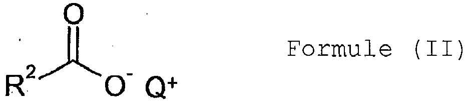 dans laquelle
R²CO a les significations de R¹ de la formule (I), et
Q⁺ a les significations de Q⁺ de la formule (I),
en des quantités inférieures ou égales à 3,0 % en poids par rapport à la composition totale, et
d) de l'eau,
e) mais ne contenant aucun solvant organique.

6. Composition selon la revendication 5, **caractérisée en ce qu'**elle contient le composant a) en des quantités de 23,0 à 27,0 % en poids par rapport à la composition totale.

7. Composition selon la revendication 5 ou 6, **caractérisée en ce que** Q⁺ représente Na⁺.

8. Composition selon l'une ou plusieurs des revendications 5 à 7, **caractérisée en ce qu'**elle contient la ou les substances Q⁺Cl⁻ en des quantités de 1,0 à 8,0 % en poids par rapport à la composition totale.

9. Composition selon l'une ou plusieurs des revendications 5 à 8, **caractérisée en ce qu'**elle contient le ou les sels d'acides gras de formule (II) en des quantités de 0,01 à 2,0 % en poids, par rapport à la composition totale.

10. Composition selon l'une ou plusieurs des revendications 5 à 9, **caractérisée en ce que** la pureté des acylglycinates qui y sont contenus de formule (I) est de 90 % ou plus, cette pureté étant rapportée à la somme du sel d'acide gras de formule (II) et de l'acylglycinate de formule (I) et étant calculée par la formule "pureté des acylglycinates = [quantité d'acylglycinate : (quantité d'acylglycinate + quantité de sel d'acide gras)]".

11. Composition selon l'une ou plusieurs des revendications 5 à 10, **caractérisée en ce qu'**elle présente une viscosité à 40°C inférieure à 5000 mPa.s.

12. Utilisation d'une composition selon l'une ou plusieurs des revendications 5 à 11 pour fabriquer une préparation cosmétique.

13. Utilisation d'une composition selon l'une ou plusieurs des revendications 5 à 11 en tant que tensioactif dans une préparation cosmétique.
